Europäisches Patentamt

**European Patent Office**

Office européen des brevets

(19)

(11) Publication number: **0 228 826**
**B1**

(12)                           **EUROPEAN PATENT SPECIFICATION**

(45) Date of publication of the patent specification:
04.07.90

(51) Int. Cl.⁵: **A61M 25/02**

(21) Application number: **86309447.0**

(22) Date of filing: **04.12.86**

(54) Fixing device for nasogastric catheter and catheter assembly.

(30) Priority: **09.12.85 JP 189202/85 U**

(43) Date of publication of application:
**15.07.87 Bulletin 87/29**

(45) Publication of the grant of the patent:
**04.07.90 Bulletin 90/27**

(84) Designated Contracting States:
**AT BE CH DE ES FR GB IT LI LU NL SE**

(56) References cited:
**EP-A- 0 021 446**
**JP-A-60 222 067**
**US-A- 4 114 626**
**US-A- 4 120 304**
**US-A- 4 351 331**

(73) Proprietor: **SHERWOOD MEDICAL COMPANY,**
**1831 Olive Street, St. Louis, MO 63103(US)**

(72) Inventor: **Okada,Yosuke, 2748-1, Ichinomiya, Morimachi**
**Shuchi-Gun, Shizuoka-ken(JP)**

(74) Representative: **Porter, Graham Ronald et al, c/o Wyeth**
**Laboratories Huntercombe Lane South, Taplow**
**Maidenhead Berkshire SL6 0PH(GB)**

ACTORUM AG

## Description

This invention relates to fixing device for a catheter for nasogastric intubation and an assembly of such a device, a catheter and a sheath.

Normally, a nasogastric catheter for supplying nutrition comprises a small diameter, soft plastics tube, having a weight sealed in the distal end thereof. The tube is inserted through the nose and the weighted end assists the tube into stomach or the intestine under the action of gravity. Food is supplied through the tube to one or two side holes positioned just proximally of the weight.

Nasogastric catheters may be kept in place for long periods of time and this is painful to the patient. To relieve the pain, it is preferable that the catheter is formed of a material as soft as possible.

However, since a soft plastics tube lacks stiffness, it is difficult to pass the tube through the nostril, larynx and oesophagus, and then the convoluted stomach and intestinal passage.

For this reason, in conventional method, a guide wire is inserted into the bore of the catheter to increase stiffness during the catheter insertion process. However the guide wire or catheter must be lubricated to decrease the frictional resistance therebetween and in addition insertion of the guide wire is cumbersome. There is also a danger that the end of the guide wire may emerge through the side holes of the catheter and pierce the walls of internal organs.

It is undesirable to make the catheter of hard plastics for, notwithstanding patient pain, long term intubation can cause damage to body tissue in contact with the catheter.

It has been proposed to provide a relatively stiff sheath for the catheter to aid insertion. One known sheath includes longitudinal split lines and is used in conjunction with a fixing device which retains the catheter in place whilst the sheath is withdrawn. The known fixing device is described in Japanese Patent Application No. 79178/1984 (published as JP-A-60 222 067) and comprises a body having a groove into which a catheter may be snapped, the wall of the groove thereby pinching the catheter firmly. The sleeve is drawn through the fixing device and separated from the catheter.

This device has the disadvantage that if the catheter remains in the groove for a long period, for example between manufacture and use, it causes deformation which either reduces the pinching power of the groove or squeezes the wall of the catheter which reduces the cross-sectional area thereof.

Furthermore, the prior art fixing device allows undesirable rotation of the sheath and/or catheter as the sheath is removed.

The present invention aims to overcome the disadvantages described above. According to the invention there is provided a fixing device for a nasogastric catheter and comprising a base with an aperture therethrough, a bridge on the base having two holes in the span thereof and a slot through the base and the bridge connecting said aperture and holes to the exterior of the device, one of said holes being of larger diameter than the other.

The larger hole in the bridge span is sized to loosely locate a nasogastric catheter whilst the smaller hole is sized to grip the catheter against movement therethrough; the slot permits the catheter to be squeezed into one or other of the holes. Preferably the inner hole is larger than the outer hole.

The invention also provides a nasogastric catheter assembly comprising the fixing device aforesaid, a catheter and a catheter sheath splittable longitudinally into two substantially equal parts, the sheath passing through the aperture and the proximal end of said sheath being split to emerge through opposite portals of the bridge.

In this arrangement the sheath parts emerge through defined openings in the device and the troublesome rotation observed in the prior art device is obviated.

During manufacture of an assembly, the catheter is located in the larger hole of the bridge span. Accordingly the fixing device is unstressed during storage and the catheter is not squeezed. When it is desired to use the assembly the catheter may be snapped into the smaller hole for retention during insertion and withdrawal of the sheath; thereafter the catheter may be snapped back into the larger hole, or the fixing device removed for disposal. The fixing device may be retained for use in a subsequent nasogastric catheter insertion procedure using a new catheter and sheath. The proximal end of the catheter is connected to a liquid food supply.

The inner wall of the sheath and outer wall of the catheter preferably have a matt finish which improves the sliding properties thereof; alternatively a lubricant can be used.

Other features of the invention will be apparent from the following description of a preferred embodiment shown by way of example only in the accompanying drawings in which:-

Figure 1 shows a catheter for nasogastric intubation and incorporating a fixing device according to the present invention;

Figures 2 and 3 are enlarged explanatory views of the fixing device showing stages in the use thereof;

Figure 4 illustrates a stage in the insertion process of a nasogastric catheter; and

Figure 5 shows a prior art fixing device.

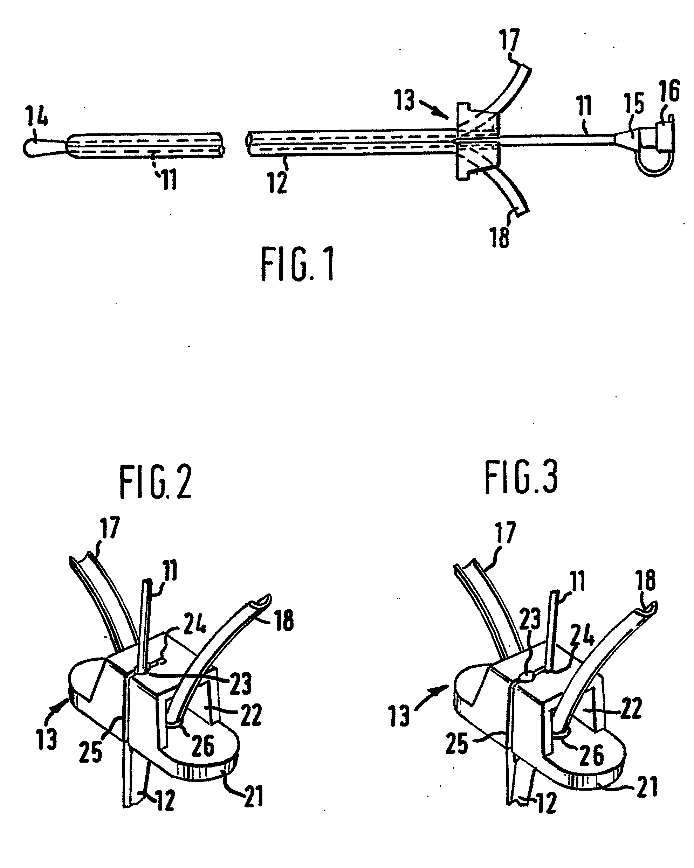

With reference to Figure 1 there is shown a catheter assembly comprising a catheter 11, sheath 12 and fixing device 13. The catheter 11 is of very soft plastic and is inserted in a plastic sheath 12 of a material having slightly greater elasticity and rigidity than the catheter. A weight 14 is encased in the distal end of the catheter and a side hole or holes (not shown) for supplying food are provided just proximally of the weight 14. An end fitting 15 for connection to a food supply is closed by a cap 16. The sheath tube 12 is weakened longitudinally either by a decreased wall thickness or by a line of generally incompatible material. In the preferred embodiment the sheath is splittable into two halves and the free ends 17, 18 of the sheath protrude through the fixing device as shown.

The fixing device 13 is shown in Figures 2 and 3 and comprises a base plate 21 having an upstanding bridge 22. Two holes 23, 24 are provided in the span of the bridge 22 and are connected by a slot 25 extending through the baseplate and one wall of the bridge. The outer hole 23 is larger than the inner hole 24. The baseplate has an aperture 26 therethrough.

Use of the fixing device is as follows. The catheter 11 is inserted into the stomach using the relatively stiff sheath 12. The catheter passes through the baseplate aperture 26 and the larger hole 23; the hole 23 is sized to allow free movement of the catheter. The sheath 12 also passes through the base aperture 26 and the proximal end thereof is split, each part of the sheath emerging through a respective portal of the bridge 22.

When the catheter is inserted to the required depth, which may be confirmed by x-ray for example, the catheter is snapped through the groove into the smaller hole 24 which is sized to grip the catheter against axial movement.

The sheath is then drawn through the fixing device for disposal; the catheter being securely held by the wall of the hole 24. The two parts of the sheath are pulled in approximately opposite directions and the reaction force urges the fixing device against the patient's nose thereby retaining the catheter in place. The assembly thus allows a single person to instal a nasogastric catheter.

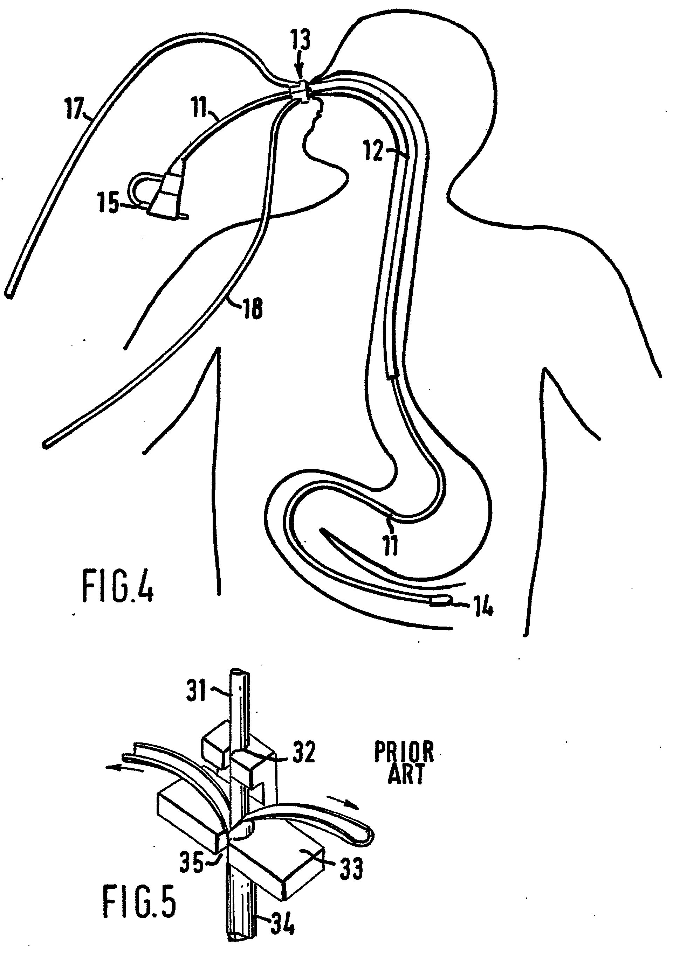

A partially withdrawn sheath is depicted in Figure 4 of the drawings. Once the sheath is withdrawn the catheter may be moved back into the larger hole 23, where it is loosely retained, or the fixing device may be removed for disposal. The end fitting 15 is connected to a source of food or medication as required.

It may be advantageous to snap the catheter 11 into the smaller hole 24 during the insertion process.

Figure 5 shows a prior art device in which the catheter 31 is snapped into a hole 32 in a fixing device 33 for retention against movement therethrough. The free ends of a splittable sheath 34 emerge through an aperture 35 in the base of the device 33 but are not guided.

The sheath can of course be used to introduce an unweighted catheter into the body because the sheath has the necessary rigidity and flexibility.

It is not entirely necessary that the sheath be splittable into two parts; a sheath with a single longitudinal weakened portion will also work.

The applicants do not intend the scope of the invention to be limited by the above description of a preferred embodiment but only by the claims appended hereto.

## Claims

1. A fixing device for a nasogastric catheter and comprising a baseplate (21) with an aperture (26) therethrough, a bridge (22) on the baseplate having two holes (23, 24) in the span thereof and a slot (25) connecting said aperture (26) and holes (23, 24) to the exterior of the device, one of said holes being of larger diameter than the other.

2. A fixing device according to Claim 1, wherein the inner hole (24) is smaller than the outer hole (23).

3. A fixing device according to Claim 1 or Claim 2, wherein the device is an integral plastics moulding.

4. A fixing device according to any preceding claim, wherein the slot (25) is perpendicular to said baseplate (21).

5. A fixing device according to any preceding claim, wherein the slot (25) is perpendicular to the axis of said bridge (22).

6. A fixing device according to any preceding claim, wherein the slot (25) passes through the centre of said holes (23, 24) and aperture (26).

7. A nasogastric catheter assembly comprising a fixing device according to any preceding claim, a catheter (11) and a catheter sheath (12), splittable into two parts, the sheath (12) passing through the aperture (26) of said fixing device and the proximal end of said sheath (12) being split to emerge through opposite portals of the bridge (22) of said device.

8. An assembly according to Claim 7, wherein the proximal end of said catheter (11) emerges through the larger hole (23) of said device.

## Patentansprüche

1. Feststellvorrichtung für Nasenmagenkatheter mit einer Grundplatte (21) und einer darin ausgebildeten Öffnung (26), einer Brücke (22) auf der Grundplatte, mit zwei Bohrungen (23, 24) und einem Schlitz (25), welcher die Öffnung (26) und die Bohrungen (23, 24) mit der Umgebung der Vorrichtung verbindet, wobei eine der Bohrungen einen größeren Durchmesser als die andere aufweist.

2. Feststellvorrichtung nach Anspruch 1, worin die innere Bohrung (24) kleiner ist als die äußere Bohrung (23).

3. Feststellvorrichtung nach Anspruch 1 oder Anspruch 2, worin die Vorrichtung ein einteiliges Kunststoff-Formteil ist.

4. Feststellvorrichtung nach einem der vorhergehenden Ansprüche, worin der Schlitz (25) senkrecht zur Grundplatte (21) ausgebildet ist.

5. Feststellvorrichtung nach einem der vorhergehenden Ansprüche, worin der Schlitz (25) senkrecht zur Achse der Brücke (22) ausgebildet ist.

6. Feststellvorrichtung nach einem der vorhergehenden Ansprüche, worin der Schlitz (25) durch das Zentrum der Bohrungen (23, 24) und der Öffnung (26) verläuft.

7. Nasenmagenkatheteranordnung mit einer Feststellvorrichtung nach einem der vorhergehenden Ansprüche, einem Katheter (11) und einer Katheterhülle (12), welche in zwei Hälften trennbar ist, wobei die Hülle (12) die Öffnung (26) der Feststellvorrichtung durchläuft und das proximale Ende der Hülle (12) aufgetrennt ist, um durch die gegenüberliegenden Eingänge der Brücke (22) der Vorrichtung auszutreten.

8. Anordnung nach Anspruch 7, worin das proximale Ende des Katheters (11) durch die größere Öffnung (23) der Vorrichtung austritt.

## Revendications

1.- Dispositif de fixation pour un cathéter nasogastrique comprenant une plaque de base (21) percée d'une ouverture (26), un pont (22) sur la plaque de base comportant deux forures (23, 24) dans sa partie supérieure et une fente (25) faisant communiquer l'ouverture (26) et les forures (23, 24) avec l'extérieur du dispositif, une des forures étant de plus grand diamètre que l'autre.

2.- Dispositif de fixation suivant la revendication 1, dans lequel la forure intérieure (24) est plus petite que la forure extérieure (23).

3.- Dispositif de fixation suivant la revendication 1 ou 2, qui est formé d'une seule pièce moulée en matière plastique.

4.- Dispositif de fixation suivant l'une quelconque des revendications précédentes, dans lequel la fente (25) est perpendiculaire à la plaque de base (21).

5.- Dispositif de fixation suivant l'une quelconque des revendications précédentes, dans lequel la fente (25) est perpendiculaire à l'axe du pont (22).

6.- Dispositif de fixation suivant l'une quelconque des revendications précédentes, dans lequel la fente (25) passe par le centre des forures (23, 24) et de l'ouverture (26).

7.- Ensemble de cathéter nasogastrique comprenant un dispositif de fixation suivant l'une quelconque des revendications précédentes, un cathéter (11) et une gaine de cathéter (12) pouvant être divisée en deux parties, la gaine (12) traversant l'ouverture (26) du dispositif de fixation et l'extrémité proximale de la gaine (12) étant divisée de manière à sortir par des entrées opposées du pont (22) du dispositif.

8.- Ensemble suivant la revendication 7, dans lequel l'extrémité proximale du cathéter (11) sort par la grande forure (23) du dispositif.

FIG. 1

FIG. 2

FIG. 3

FIG.4

FIG.5

PRIOR ART